# EUROPEAN PATENT SPECIFICATION

(11) **EP 1 289 479 B1**
(45) Date of publication and mention of the grant of the patent: **08.11.2006**
(21) Application number: 01951550.1
(22) Date of filing: 30.05.2001
(51) Int. Cl.: A61Q 5/12, A61K 8/31, A61K 8/37, A61P 31/10

(54) **HAIR OILS**
HAARÖLE
HUILES CAPILLAIRES

(30) Priority: 13.06.2000 GB 0014426
(43) Date of publication of application: 12.03.2003
(73) Proprietor: UNILEVER PLC, London EC4P 4BQ (GB); UNILEVER N.V., 3013 AL Rotterdam (NL)
(72) Inventor: DICKINSON, Kelvin B., Unilever Res. Port Sunlight, Wirral, Merseyside CH63 3JW (GB); EVERAERT, E.P.J.M., Unilever Res. Port Sunlight, Wirral, Merseyside CH63 3JW (GB); TAN-WALKER, Ruby L.B., Unilever Res. Port Sunlight, Wirral, Merseyside CH63 3JW (GB)
(74) Representative: Whaley, Christopher
(86) International application number: PCT/EP2001/006269
(87) International publication number: WO 2001/095866

(56) References cited:
- EP-A- 0 546 235
- CH-A- 272 709
- DE-B- 1 035 855
- FR-A- 838 699
- FR-A- 1 089 353
- GB-A- 564 551
- GB-A- 824 353
- US-A- 3 932 611
- US-A- 4 849 214
- DATABASE PROMT [Online] STN INTERNATIONAL; AN 2000:482700, "Body: Massage oil " XP002180877 & ADVANSTAR COMMUNICATIONS, INC.: GLOBAL COSMETIC INDUSTRY, vol. 166, no. 5, May 2000 (2000-05), page 45
- DATABASE PROMT [Online] STN INTERNATIONAL; AN 2000:282723, "Parachute dandruff solution coconut hair oil" XP002180876 & MARKETING INTELLIGENCE SERVICE LTD.: INTERNATIONAL PRODUCT ALERT, vol. 17, no. 6, 20 March 2000 (2000-03-20),
- NN: "Paraffin(öl)" RÖMPP ONLINE, [Online] 2005, pages 1-2, Retrieved from the Internet: URL:http://www.roempp.com/prod/roempp.php> [retrieved on 2005-08-18]

## Description

### FIELD OF THE INVENTION

This invention relates to hair oils which incorporate a specific blend of oil types and which have enhanced sensory properties.

### BACKGROUND OF INVENTION AND PRIOR ART

Consumers oil hair both pre wash and post wash. Pre wash oiling is done as it is believed that oils nourish hair and protect it during the wash process. Post wash oiling is done for manageability and styling. The oiling habit is widely practised by around 800 million people across the Central Asia and Middle East region.

Coconut oil is by far the most common oil used in the Central Asia and Middle East region for hair care. It offers a high level of conditioning benefits, but with the drawback of greasy feel.

CH272709 discloses sprayable hair oils on the basis of mineral oils and glyceride fatty esters.

The present inventors have found that hair oils which incorporate a specific blend of oil types can deliver an equivalent level of conditioning benefits to coconut oil, but with superior sensory properties, in particular less greasy feel. A further advantage associated with hair oils according to the invention is enhanced penetration into the hair fibre.

### DEFINITION OF THE INVENTION

The present invention provides a hair oil comprising a blend of:
(i) from 20% to 80%, by weight based on total weight, of a first oily component which is one or more glyceride fatty esters, and
(ii) from 20% to 80%, by weight based on total weight, of a second oily component which is a hydrocarbon oil selected from a light mineral oil, straight chain hydrocarbon oils containing 6 to 16 carbon atoms, branched chain hydrocarbon oils containing from 6 to 20 carbon atoms, and blends thereof.

### DETAILED DESCRIPTION OF THE INVENTION

### (i) Glyceride Fatty Ester

By "glyceride fatty esters" is meant the mono-, di-, and tri-esters formed between glycerol and long chain carboxylic acids such as C₆-C₃₀ carboxylic acids. The carboxylic acids may be saturated or unsaturated or contain hydrophilic groups such as hydroxyl.

Preferred glyceride fatty esters are derived from carboxylic acids of carbon chain length ranging from C₆ to C₂₄, preferably C₁₀ to C₂₂, most preferably C₁₂ to C₁₈.

Suitable glyceride fatty esters for use in hair oils of the invention will generally have a viscosity at ambient temperature (25 to 30°C) of from 0.01 to 0.8 Pa.s, preferably from 0.015 to 0.6 Pa.s, more preferably from 0.02 to 0.065 Pa.s as measured by a Carri-Med CSL2 100 controlled stress rheometer, from TA Instruments Inc., New Castle, Delaware (USA).

A variety of these types of materials are present in vegetable and animal fats and oils, such as camellia oil, coconut oil, castor oil, safflower oil, sunflower oil, peanut oil, cottonseed oil, corn oil, olive oil, cod liver oil, almond oil, avocado oil, palm oil, sesame oil, lanolin and soybean oil. These have various ranges of carbon chain lengths depending on the source, typically between about 12 to about 18 carbon atoms. Synthetic oils include trimyristin, triolein and tristearin glyceryl dilaurate. Vegetable derived glyceride fatty esters are particularly preferred, and specific examples of preferred materials for inclusion in hair oils of the invention as sources of glyceride fatty esters include almond oil, castor oil, coconut oil, sesame oil, sunflower oil and soybean oil. Coconut oil, sunflower oil, almond oil and mixtures thereof are particularly preferred.

The glyceride fatty ester may be present in hair oils of the invention as a single material or as a blend.

The total content of glyceride fatty ester in hair oils of the invention suitably ranges from 10% to 95%, preferably from 20% to 80%, by weight based on total weight of the hair oil.

### (ii) Hydrocarbon Oil

Suitable hydrocarbon oils include straight chain aliphatic hydrocarbons (saturated or unsaturated), and branched chain aliphatic hydrocarbons (saturated or unsaturated). Suitable Straight chain hydrocarbon oils contain from 6 to 16 carbon atoms, preferably from 8 up to 14 carbon atoms. Branched chain hydrocarbon oils can and typically may contain higher numbers of carbon atoms, e.g. from about 6 up to about 20 carbon atoms, preferably from about 8 up to about 18 carbon atoms.

Suitable hydrocarbon oils of the invention will generally have a viscosity at ambient temperature (25 to 30°C) of from 0.0001 to 0.05 Pa.s, more preferably from 0.001 to 0.02 Pa.s as measured by a Carri-Med CSL2 100 controlled stress rheometer, from TA Instruments Inc., New Castle, Delaware (USA).

A preferred hydrocarbon oil is light mineral oil. Mineral oils are clear oily liquids obtained from petroleum oil, from which waxes have been removed, and the more volatile fractions removed by distillation. The fraction distilling between 250°C to 300°C is termed mineral oil, and it consists of a mixture of hydrocarbons, in which the number of carbon atoms per hydrocarbon molecule generally ranges from C₁₀ to C₄₀. Mineral oil may be characterised in terms of its viscosity, where light mineral oil is relatively less viscous than heavy mineral oil, and these terms are defined more specifically in the U.S. Pharmacopoeia, 22nd revision, p. 899 (1990). A commercially available example of a suitable light mineral oil for use in the invention is Sirius M40 (carbon chain length C₁₀-C₂₈, mainly C₁₂-C₂₀, viscosity 4.3 x 10⁻³ Pa.s), available from Silkolene.

Other hydrocarbon oils that may be used in the invention include relatively lower molecular weight hydrocarbons including linear saturated hydrocarbons such a tetradecane, hexadecane, and octadecane, cyclic hydrocarbons such as dioctylcyclohexane (e.g. CETIOL S from Henkel), branched chain hydrocarbons (e.g. ISOPAR L and ISOPAR V from Exxon Corp.).

The hydrocarbon oil may be present in hair oils of the invention as a single material or as a blend.

The total content of hydrocarbon oil in hair oils of the invention suitably ranges from 5% to 90%, preferably from 20% to 80%, by weight based on total weight of the hair oil.

The glyceride fatty ester:hydrocarbon oil weight ratio in hair oils of the invention may suitably range from 95:5 to 5:95, preferably from 90:10 to 10:90, most preferably from 80:20 to 20:80. Particularly preferred are blends of [coconut oil and/or sunflower oil and/or almond oil] and light mineral oil, in which the [coconut oil and/or sunflower oil and/or almond oil]:light mineral oil weight ratio is 60:40.

### Other Oily Materials

Other oily materials may also be present in combination with the hydrocarbon oils and glyceride fatty esters in hair oils of the invention. Suitable additional oily materials include other fatty esters, fatty alcohols and fatty ethers.

In general, fatty esters, fatty alcohols and ethers are characterised by having at least 10 carbon atoms, and include esters and ethers with hydrocarbyl chains derived from fatty acids or alcohols, e.g., monocarboxylic acid esters, polyhydric alcohol esters, and di- and tricarboxylic acid esters and ethers, and fatty alcohols with a carbon chain carbon chain length of between 10 and 18, e.g. lauryl alcohol, cetyl alcohol or cetostearyl alcohol.

Examples include isopropyl myristate, isopropyl palmitate, cetearyl isononanoate, cetearyl octanoate, diethylene glycol monoethyl ether oleate, dicaprylyl ether, caprylic acid/capric acid propylene glycol diester and mixtures of any of the above.

The total content of other oily material in hair oils of the invention suitably ranges from 0.01% to 50%, preferably from 1.0% to 20%, by weight based on total weight of the hair oil.

### Product Form

Compositions of this invention are preferably in anydrous form, i.e. used as neat hair oil.

### Optional Ingredients

Compositions of this invention may contain any other ingredient normally used in hair treatment formulations. These other ingredients may include, viscosity modifiers, preservatives, colouring agents, polyols such as glycerine and polypropylene glycol, chelating agents such as EDTA, antioxidants such as BHT (butylhydroxytoluene), vitamin E acetate, fragrances, antimicrobials and sunscreens and lipid soluble ingredients e.g. fatty acids or sterols. Each of these ingredients will be present in an amount effective to accomplish its purpose. Generally these optional ingredients are included individually at a level of up to about 5% by weight of the total hair oil.

The invention is further illustrated by way of the following Examples, in which all percentages are by weight based on total weight unless otherwise stated.

### EXAMPLES

| **Formulation Examples:** | | | | | |
|---|---|---|---|---|---|
| | | | | | |
| **Ingredient** | Control/ Benchmark | Example 1 | Example 2 | Example 3 | Example 4 |
| | | | | | |

| *Triglycerides* | | | | | |
|---|---|---|---|---|---|
| Coconut oil | 100% | 60% | 60% | 60% | 60% |
| Castor oil | - | 5% | - | - | - |
| Almond oil | - | - | - | 5% | - |
| Sunflower oil | - | - | 5% | - | - |
| | | | | | |

| *Hydrocarbon oils* | | | | | |
|---|---|---|---|---|---|
| Light mineral oil | - | 20% | 35% | 35% | 40% |
| | | | | | |

| *Other oily materials* | | | | | |
|---|---|---|---|---|---|
| Isopropyl palmitate | - | 15% | - | - | - |
| | 100% | 100% | 100% | 100% | 100% |
| | | | | | |

| **Formulation Performances** | | | | | |
|---|---|---|---|---|---|
| Greasy feel | 72.8 | 61.7 | 58.9 | 54.7 | 61.1 |
| Ease of combing | 74.8 | 75.6 | 70.4 | 74.7 | 70.3 |
| | | | | | |

The formulation performances such as greasy feel and ease of combing were evaluated using sensory panels. Results are give in normalised values. A preferred formulation will have similar "ease-of-combing" as the control/benchmark coconut oil and a significantly lower "greasy feel".

Oil penetration into the hair fibre was measured using fluorescence techniques on oiled hair cross-sections using a lipid soluble fluorescent dye. Results showed that mixing light hydrocarbon oil to the hair oils improves significantly the total oil penetration into the hair fibre. Data are given in the following Table:

| | **Retained fluorescence intensity** |
|---|---|
| Non oiled hair | 0 cps |
| control/benchmark | 1722 cps |
| Example 3 | 110965 cps |

## Claims

1. A hair oil comprising a blend of:
(i) from 20% to 80%, by weight baaed on total weight, of a first oily component which is one or more glyceride fatty, esters, and
(ii) from 20% to 80%, by weight baaed on total weight, of a second oily component which is a hydrocarbon oil selected from a light mineral oil, straight chain hydrocarbon oils containing from 6 to 16 carbon atoms, branched-chain hydrocarbon oils containing from 6 to 20 carbon atoms and blends thereof.

2. A hair oil according to claim 1, in which the source of glyceride fatty esters is selected from coconut oil, sunflower oil, almond oil and mixtures thereof.

3. A hair oil according to claim 1 or 2, in which the glyceride fatty ester:hydrocarbon oil weight ratio ranges from 80:20 to 20:80.

4. A hair oil according to any one of claims 1 to 3, which is in anhydrous form.

## Patentansprüche

1. Haaröl, umfassend ein Gemisch von:
(i) 20 % bis 80 Gew.-%, bezogen auf das Gesamtgewicht, von einer ersten öligen Komponente, die einen oder mehrere Glyceridfettester darstellt, und
(ii) 20 % bis 80 Gew.-%, bezogen auf das Gesamtgewicht, von einer zweiten öligen Komponente, die ein Kohlenwasserstofföl darstellt, ausgewählt aus einem leichten Mineralöl, geradkettigen Kohlenwasserstoffölen, die 6 bis 16 Kohlenstoffatome enthalten, verzweigtkettigen Kohlenwasserstoffölen, die 6 bis 20 Kohlenstoffatome enthalten, und Gemischen davon.

2. Haaröl nach Anspruch 1, worin die Quelle von Glyceridfettestern aus Kokosnussöl, Sonnenblumenöl, Mandelöl und Gemischen davon ausgewählt ist.

3. Haaröl nach Anspruch 1 oder 2, worin das Gewichtsverhältnis von dem Glyceridfettester : Kohlenwasserstofföl im Bereich von 80:20 bis 20:80 liegt.

4. Haaröl nach einem der Ansprüche 1 bis 3, welches in wasserfreier Form vorliegt.

## Revendications

1. Huile capillaire comprenant un mélange de :
(i) de 20 % à 80 %, en poids par rapport au poids total, d'un premier composant huileux qui est un ou plusieurs esters gras de glycérides, et
(ii) de 20 % à 80 %, en poids par rapport au poids total, d'un second composant huileux qui est une huile hydrocarbonée choisie parmi une huile minérale légère, des huiles hydrocarbonées à chaîne linéaire contenant de 6 à 16 atomes de carbone, des huiles hydrocarbonées à chaîne ramifiée contenant de 6 à 20 atomes de carbone et des mélanges de celles-ci.

2. Huile capillaire selon la revendication 1, dans laquelle la source d'ester gras de glycéride est choisie parmi l'huile de coco, l'huile de tournesol, l'huile d'amande et les mélanges de celles-ci.

3. Huile capillaire selon la revendication 1 ou 2, dans laquelle le rapport en poids de l'ester gras de glycéride/l'huile hydrocarbonée se trouve dans la plage de 80/20 à 20/80.

4. Huile capillaire selon l'une quelconque des revendications 1 à 3, qui est sous une forme anhydre.
